# EUROPEAN PATENT APPLICATION

(11) **EP 4 644 572 A1**
(43) Date of publication of application: **05.11.2025**
(21) Application number: 24173026.6
(22) Date of filing: 29.04.2024
(51) Int. Cl.: C12Q 1/6895

(54) **METHOD FOR ANALYSING TRAIT PURITY IN LARGE SEED BULKS**

(71) Applicant: KWS SAAT SE & Co. KGaA, 37555 Einbeck (DE)
(72) Inventor: Knaak, Carsten, 37079 Göttingen (DE); Braatz, Janina, 37574 Einbeck (DE); Habekost, Sandra, 37586 Dassel (DE); Kloiber-Maitz, Monika, 37574 Einbeck (DE); Grundmann, Olav, 28357 Bremen (DE)

(57) **Abstract**

The present invention relates to the detection of contamination with wild-type (WT) probes in large pools of probes, for example seed lots, using amplification (e.g. ddPCR) with primers and probe specific for the WT and primers and probe specific for a genetic modification (GM).

## Description

### Technical Field

The present invention relates to the detection of contamination with wild-type (WT) probes in large pools of probes, for example seed lots.

### Background

Transgenic genetically modified plants underly various regulations worldwide, including but not limited to prevention of uncontrolled spread. It is common practice to test plant tissues for presence or seed batches for contamination with undesired transgenic genetically modified plants. Detection methods are for example described in Delobel et al. (2011) and Long et al. (2017). However, when transgenic genetically modified plants are produced on commercial level, additionally the absence of the transgenic event of interest is of high relevance. Even few plants lacking the transgenic event of interest in a production field intended to multiply plants comprising said transgenic event of interest can cause wide-spread trait loss in the following generations. Depending on the nature of the transgenic event of interest, the consequence can for example range from unexpected herbicide sensitivity to vulnerability to insects. Selling seed lots of plants comprising a transgenic event of interest contaminated with plant seeds lacking the transgenic event of interest can lead to yield losses for the farmer and damage the reputation of the seed company.

The problem of contamination of seed lots comprising a certain genetic modification (GM) with seeds, lacking the respective GM is not restricted to seed lots comprising transgenic events but to any seed lot or seed batch amplified for sale. The GM of interest in the seeds amplified for sale may comprise for example a beneficial natural mutation, a GM introduced into the plant using random mutagenesis or genome editing technologies, beneficial genes inbred from distantly related plants and the like.

The common detection methods designed for testing for the presence of a GM are not sensitive enough to identify its absence in large seed batches. The method described herein makes marker analysis for this purpose fast, reliable, highly sensitive and economically feasible. Without this invention, seed bulks could in some cases be tested for trait purity by spraying tests. However, this requires time and effort for plant germination and spraying. Traits other than herbicide tolerance are practically not possible to score phenotypically in large volumes. The invention allows for a simplified and advanced quality management of GM lines. Seed increases can be tested for trait purity at high sensitivity and resources can be saved when trait loss is observed. Another advantage of the high sensitivity is that trait purity analysis and adventitious presence (AP) testing can be run on the same sample. AP testing underlies governmental regulation in different countries and can be mandatory by law. Such a sample can serve a dual purpose with valuable information gain at comparably low additional cost and workload.

### Summary of the Invention

A first aspect of the invention is a method for quantifying in a population or batch of microorganisms, plants, plant seeds, tissues and/or cells comprising a genetic modification (GM) the occurrence of microorganisms, plants, plant seeds, tissues and/or cells lacking said GM comprising the steps of:
a. providing a first set of primers and probe specific for the absence of said GM, and
b. providing a second set of primers and probe specific for the presence of said GM, and
c. providing a pool of nucleic acid molecules comprising an equal amount of nucleic acid molecules derived from each individual in said population or batch to be assessed, and
d. performing an amplification reaction using said first and second set of primers and probes on the pool of nucleic acid molecules of step c, and
e. detecting the signals generated by the amplification reactions of said first and second set of primers and probes, and
f. calculating the ratio of absence to presence signals,
thereby quantifying the occurrence of microorganisms, plants, plant seeds, tissues and/or cells lacking said GM in said population or batch to be assessed.

Calculation of the ratio of absence or presence of the signal is for example described in BioRad Bulletin 6407 "Droplet Digital PCR" (https://www.bio-rad.com/webroot/web/pdf/lsr/literature/Bulletin_6407.pdf), in Beltz et al. 2018, Advances in Bioscience and Biotechnology, 9 (9) (DOI: 10.4236/abb.2018.99034) or in "Verification of analytical methods for GMO testing when implementing interlaboratory validated methods" (https://gmo-crl.jrc.ec.europa.eu/doc/ENGL%20MV%20WG%20Report%20July%202011.pdf).

The first and second set of primers are specific for the region of up to 1000, preferably up to 800, more preferably up to 500, more preferably up to 300, most preferably up to 250 nucleotides upstream and/or downstream of the locus of said GM.

The GM may be any genetic modification such as a transgene, a natural mutation, a mutation introduced by random mutagenesis or genome editing, any insertion, deletion, inversion or rearrangement in the genome, an additional gene or genetic region, a QTL and the like. The mutation may be a point-mutation. The mutation may further be a deletion of one or more bases or an insertion of one or more bases, herein together referred to as InDel.

The pool of nucleic acid molecules comprising an equal amount of nucleic acid molecules derived from each individual in said population or batch to be assessed may be produced by extracting nucleic acid molecules from the entire pool of individuals, by extracting nucleic acid molecules of sub pools of the entire pool of individuals and subsequent pooling such sub pools or by extracting nucleic acid molecules from each individual member of the population to be assessed and subsequent pooling of the nucleic acid molecules, in the latter case preferably by pooling equal amounts of nucleic acids from each individual of the population. Also, the nucleic acids may be extracted from the complete individual comprised in the pool or batch or from parts taken from each individual comprised in the pool or batch.

A further aspect of the invention is the method as defined above, wherein the first set of primers and probes comprises at least two forward and/or reverse primers specific for the region of up to 1000, preferably up to 800, more preferably up to 500, more preferably up to 300, most preferably up to 250 nucleotides upstream and/or downstream of the locus of said GM of various microorganisms, plants, plant seeds, tissues and/or cells lacking said GM. Microorganisms, plants, plant seeds, tissues and/or cells lacking a GM of interest may be addressed herein as wild-type. Wild-type may for example be a plant line used as starting material for introducing the respective genetic modification or any genotype of a certain plant species not comprising the respective genetic modification.

A further aspect of the invention is the method as defined above, wherein the second set of primers and probes comprises at least two probes detecting distinct variants of the GM.

A further aspect of the invention is the method as defined above, wherein the probes are fluorescent probes.

A further aspect of the invention is the method as defined above, wherein the amplification reaction is a ddPCR, a qPCR or a rhAMP PCR. In case the GM is a transgene or a larger insertion or inversion, preferably ddPCR is applied. In case the GM is a point-mutation or an InDel of one or few bases, preferably rhAMP PCR is applied. Few bases is to be understood as 20 or less, for example 15 or less, preferably 10 or less, more preferably 5 or less, for example 4, 3 or 2 bases.

A preferred aspect of the invention is a method for quantifying in a batch of plant seeds comprising a genetic modification (GM) a contamination with plant seeds lacking said GM comprising the steps of:
a. providing a first set of primers and probe specific for the absence of said GM, and
b. providing a second set of primers and probe specific for the presence of said GM, and
c. providing a pool of nucleic acid molecules comprising an equal amount of nucleic acid molecules derived from each individual seed in said batch to be assessed, and
d. performing an amplification reaction using said first and second set of primers and probes on the pool of nucleic acid molecules of step c, and
e. detecting the signals generated by the amplification reactions of said first and second set of primers and probes, and
f. calculating the ratio of absence to presence signals,
thereby quantifying in said batch of plant seeds the contamination with plant seeds lacking said GM.

The terms seed lot and seed batch are used herein interchangeably.

The GM may be any genetic modification such as a transgene, a natural mutation, a mutation introduced by random mutagenesis or genome editing, any insertion, deletion, inversion or rearrangement in the genome, an additional gene or genetic region, a QTL and the like. The mutation may be a point-mutation. The mutation may further be a deletion of one or more bases or an insertion of one or more bases, herein together referred to as InDel.

The first and second set of primers are specific for the region of up to 1000, preferably up to 800, more preferably up to 500, more preferably up to 300, most preferably up to 250 nucleotides upstream and/or downstream of the locus of said GM.

The pool of nucleic acid molecules comprising an equal amount of nucleic acid molecules derived from each individual in said population or batch to be assessed may be produced by extracting nucleic acid molecules from the entire pool of individuals, by extracting nucleic acid molecules of sub pools of the entire pool of individuals and subsequent pooling such sub pools or by extracting nucleic acid molecules from each individual member of the population to be assessed and subsequent pooling of the nucleic acid molecules, in the latter case preferably by pooling equal amounts of nucleic acids from each individual of the population. Also, the nucleic acids may be extracted from the complete individual comprised in the pool or batch or from parts taken from each individual comprised in the pool or batch.

A further aspect of the invention is the method as defined above, wherein the first set of primers and probes comprises at least two forward and/or reverse primers specific for the region of up to 1000, preferably up to 800, more preferably up to 500, more preferably up to 300, most preferably up to 250 nucleotides upstream and/or downstream of the locus of said GM of various microorganisms, plants, plant seeds, tissues and/or cells lacking said GM. Microorganisms, plants, plant seeds, tissues and/or cells lacking a GM of interest may be addressed herein as wild-type. Wild-type may for example be a plant line used as starting material for introducing the respective genetic modification or any genotype of a certain plant species not comprising the respective genetic modification.

A further aspect of the invention is the method as defined above, wherein the probes are fluorescent probes.

A further aspect of the invention is the method as defined above, wherein the amplification reaction is a ddPCR, a qPCR or a rhAMP PCR. In case the GM is a transgene or a larger insertion or inversion, preferably ddPCR is applied. In case the GM is a point-mutation or an InDel of one or few bases, preferably rhAMP PCR is applied. Few bases is to be understood as 20 or less, for example 15 or less, preferably 10 or less, more preferably 5 or less, for example 4, 3 or 2 bases.

A further aspect of the preferred invention is the method as defined above, wherein the batch comprises at least 100, preferably at least 500, more preferably at least 1000, more preferably at least 1500, more preferably at least 2000, more preferably at least 2500, most preferably at least 3000 individuals.

A further aspect of the preferred invention is the method as defined above, wherein a contamination of equal or less than 10%, preferably equal or less than 5%, more preferably equal or less than 1%, more preferably equal or less than 0.5%, more preferably equal or less than 0.25%, more preferably equal or less than 0.1%, more preferably equal or less than 0.05%, more preferably equal or less than 0.04%, even more preferably equal or less than 0.03%, for example 0.025% can be detected.

A further aspect of the preferred invention is the method as defined above, wherein a threshold of 10 seeds, preferably 9 seeds, preferably 8 seeds, preferably 7 seeds, preferably 6 seeds, preferably 5 seeds, preferably 4 seeds, preferably 3 seeds, preferably 2 seeds lacking said GM is defined to identify seed batches to be discarded. More preferably a threshold of 4-6 seeds, most preferably 5 seeds per 3000 lacking said GM is defined to identify seed batches to be discarded.

A further aspect of the preferred invention is the method as defined above, wherein the pool of nucleic acid molecules is used for screening for adventitious presence of undesired GM events.

A further aspect of the invention is a composition for quantifying in a population or batch of microorganisms, plants, plant seeds, tissues and/or cells comprising a nucleotide sequence of interest (NOI) the occurrence of microorganisms, plants, plant seeds, tissues and/or cells lacking said NOI comprising
a. a first set of primers and probe specific for the absence of the NOI, and
b. a second set of primers and probe specific for the NOI, and
c. a pool of nucleic acid molecules comprising an equal amount of nucleic acid molecules derived from each individual in said population or batch to be assessed.

The first and second set of primers are specific for the region of up to 1000, preferably up to 800, more preferably up to 500, more preferably up to 300, most preferably up to 250 nucleotides upstream and/or downstream of the locus of said GM.

A further aspect of the invention is the composition as defined above, wherein the first set of primers and probes comprises at least two forward and/or reverse primers specific for the region of up to 1000, preferably up to 800, more preferably up to 500, more preferably up to 300, most preferably up to 250 nucleotides upstream and/or downstream of the locus of said GM of various microorganisms, plants, plant seeds, tissues and/or cells lacking said GM.

A further aspect of the invention is the composition as defined above, wherein the second set of primers and probes comprises at least two probes detecting various variants of the NOI.

A further aspect of the invention is the composition as defined above, wherein the probes are fluorescent probes.

It must be noted that, as used in the specification and the appended claims, the singular forms "a," "an" and "the" include plural references unless the context clearly dictates otherwise. For example, reference to a component is intended also to include composition of a plurality of components. References to a composition containing "a" constituent is intended to include other constituents in addition to the one named. In other words, the terms "a," "an," and "the" do not denote a limitation of quantity, but rather denote the presence of "at least one" of the referenced item.

As used herein, the term "and/or" may mean "and," it may mean "or," it may mean "exclusive-or," it may mean "one," it may mean "some, but not all," and/or it may mean "both." The term "or" is intended to mean an inclusive "or."

The analysis to identify contamination with undesired transgenic plants in plant populations or seed batches is called "Adventitious Presence" (AP) testing. It includes both the analysis of conventional plant material, which should not have any transgenic element at all, and the analysis of transgenic material, which should only contain the intended transgenic elements but no additional transgenic elements.

Also, in describing the exemplary embodiments, terminology will be resorted to for the sake of clarity. It is intended that each term contemplates its broadest meaning as understood by those skilled in the art and includes all technical equivalents which operate in a similar manner to accomplish a similar purpose. It is to be understood that embodiments of the disclosed technology may be practiced without these specific details. In other instances, well-known methods, structures, and techniques have not been shown in detail in order not to obscure an understanding of this description. References to "one embodiment," "an embodiment," "example embodiment," "some embodiments," "certain embodiments," "various embodiments," etc., indicate that the embodiment(s) of the disclosed technology so described may include a particular feature, structure, or characteristic, but not every embodiment necessarily includes the particular feature, structure, or characteristic. Further, repeated use of the phrase "in one embodiment" does not necessarily refer to the same embodiment, although it may.

As used herein, the term "about" should be construed to refer to both of the numbers specified as the endpoint (s) of any range. Any reference to a range should be considered as providing support for any subset within that range. Ranges may be expressed herein as from "about" or "approximately" or "substantially" one particular value and/or to "about" or "approximately" or "substantially" another particular value. When such a range is expressed, other exemplary embodiments include from the one particular value and/or to the other particular value. Further, the term "about" means within an acceptable error range for the particular value as determined by one of ordinary skill in the art, which will depend in part on how the value is measured or determined, i.e., the limitations of the measurement system. For example, "about" can mean within an acceptable standard deviation, per the practice in the art. Alternatively, "about" can mean a range of up to ±20%, preferably up to ±10%, more preferably up to ±5%, and more preferably still up to ±1% of a given value. Alternatively, particularly with respect to biological systems or processes, the term can mean within an order of magnitude, preferably within 2-fold, of a value. Where particular values are described in the application and claims, unless otherwise stated, the term "about" is implicit and in this context means within an acceptable error range for the particular value.

Throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

Similarly, as used herein, "substantially free" of something, or "substantially pure", and like characterizations, can include both being "at least substantially free" of something, or "at least substantially pure", and being "completely free" of something, or "completely pure".

By "comprising" or "containing" or "including" is meant that at least the named compound, element, particle, or method step is present in the composition or article or method, but does not exclude the presence of other compounds, materials, particles, method steps, even if the other such compounds, material, particles, method steps have the same function as what is named.

Throughout this description, various components may be identified having specific values or parameters, however, these items are provided as exemplary embodiments. Indeed, the exemplary embodiments do not limit the various aspects and concepts of the present invention as many comparable parameters, sizes, ranges, and/or values may be implemented. The terms "first," "second," and the like, "primary," "secondary," and the like, do not denote any order, quantity, or importance, but rather are used to distinguish one element from another.

It is noted that terms like "specifically," "preferably," "typically," "generally," and "often" are not utilized herein to limit the scope of the claimed invention or to imply that certain features are critical, essential, or even important to the structure or function of the claimed invention. Rather, these terms are merely intended to highlight alternative or additional features that may or may not be utilized in a particular embodiment of the present invention. It is also noted that terms like "substantially" and "about" are utilized herein to represent the inherent degree of uncertainty that may be attributed to any quantitative comparison, value, measurement, or other representation.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "50 mm" is intended to mean "about 50 mm."

It is also to be understood that the mention of one or more method steps does not preclude the presence of additional method steps or intervening method steps between those steps expressly identified. Similarly, it is also to be understood that the mention of one or more components in a composition does not preclude the presence of additional components than those expressly identified.

The materials described hereinafter as making up the various elements of the present invention are intended to be illustrative and not restrictive. Many suitable materials that would perform the same or a similar function as the materials described herein are intended to be embraced within the scope of the invention. Such other materials not described herein can include, but are not limited to, materials that are developed after the time of the development of the invention, for example. Any dimensions listed in the various drawings are for illustrative purposes only and are not intended to be limiting. Other dimensions and proportions are contemplated and intended to be included within the scope of the invention.

In accordance with the present invention there may be employed conventional molecular biology, microbiology, and recombinant DNA techniques within the skill of the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989"); DNA Cloning: A Practical Approach, Volumes I and II (D.N. Glover ed. 1985); Oligonucleotide Synthesis (M.J. Gait ed. 1984); Nucleic Acid Hybridization (B.D. Hames & S.J. Higgins eds.(1985); Transcription and Translation (B.D. Hames & S.J. Higgins, eds. (1984); Animal Cell Culture (R.I. Freshney, ed. (1986); Immobilized Cells and Enzymes (IRL Press, (1986); B. Perbal, A Practical Guide To Molecular Cloning (1984); F.M. Ausubel et al. (eds.), Current Protocols in Molecular Biology, John Wiley & Sons, Inc. (1994); Plant propagation by Tissue Culture 3rd Edition, Volume 1, The Background; George et al. 2008, among others.

### Brief Description of the Drawings

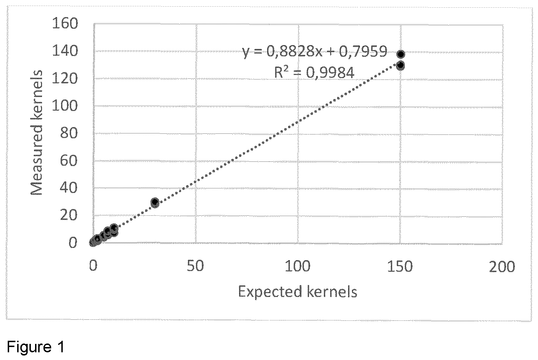

Figure 1 shows the linear correlation of expected wildtype kernels within 3,000 GM kernels vs wildtype kernels measured via ddPCR.

### Examples

The present invention is also described and demonstrated by way of the following examples. However, the use of these and other examples anywhere in the specification is illustrative only and in no way limits the scope and meaning of the invention or of any exemplified term. Likewise, the invention is not limited to any particular preferred embodiments described here. Indeed, many modifications and variations of the invention may be apparent to those skilled in the art upon reading this specification, and such variations can be made without departing from the invention in spirit or in scope. The invention is therefore to be limited only by the terms of the appended claims along with the full scope of equivalents to which those claims are entitled.

### 1. Assay design

The digital droplet PCR (ddPCR) assay was designed as a duplex marker, targeting GM event SYN-IR162-4 (MIR162) and 7 different wildtype (WT) sequence at the respective GM insertion site (4LB1913 (KWS proprietary line), 7P7010 (KWS proprietary line), 7S1434 (KWS proprietary line), B73, DK105, MO17, PH207). All wildtype sequences were considered for the assay design to detect as broad a genetic diversity as possible. Two separate reverse primers had to be used to cover sequence variation in the wildtypes. Primer WT-MIR162-R binds to a subset of wildtype plus the MIR162 material, whereas WT-Maize-Variant-R binds to another subset of wildtype lines.

**Tab. 1: Primer sequences of the WT-MIR162 ddPCR assay.**

| Primer | Orientation | Sequence (5' to 3') | Tm | % G/C | Length (bases) | SEQ ID NO |
|---|---|---|---|---|---|---|
| WT-Maize-F | Forward | TGA TGC AAA TAG GCT GGG AAT | 54 °C | 42 | 21 | 1 |
| MIR162-F | Forward | AGC GCG CAA ACT AGG ATA AAT T | 54 °C | 40 | 22 | 2 |
| WT-MIR162-R | Reverse | TGC CTT ATC TGT TGC CTT CAG A | 54 °C | 45 | 22 | 3 |
| WT-Maize-Variant-R | Reverse | GCC AGT AAA ACA ACT ACC CAT GGT | 55 °C | 45 | 24 | 4 |

Amplicon sizes were 136 bp for the wildtype and 118 bp for MIR162.

**Tab. 2: Probe sequences of the WT-MIR162 ddPCR assay.**

| Probe | Sequence (5' to 3') | Tm | % G/C | Length (bases) | SEQ ID NO |
|---|---|---|---|---|---|
| WT-Maize-FAM | 6-FAM - GAG CGC ATC GTC ACT GCT GA-BMN-Q530 | 57 °C | 60 | 20 | 5 |
| MIR162-HEX | HEX- GCG CGC GGT GTC ATC TAT GT - BMN-Q530 | 58 °C | 60 | 20 | 6 |

### 2. PCR conditions

The PCR master mix was prepared as described in Tab. 3.

**Tab. 3: PCR master mix.**

| Ingredient | Final cone. | Volume |
|---|---|---|
| 2x ddPCR Supermix for Probes (Bio-Rad) | 1x | 10 µl |
| WT-Maize-F (100 µM) | 900 nM | 0,18 µl |
| WT-Maize-R (100 µM) | 900 nM | 0,18 µl |
| WT-Maize-FAM probe (10 µM) | 200 nM | 0,40 µl |
| MIR162-F (100 µM) | 900 nM | 0,18 µl |
| MIR162-R (100 µM) | 900 nM | 0,18 µl |
| MIR162-HEX probe (10 µM) | 200 nM | 0,40 µl |
| H2Obidest | | 3,48 µl |

The final reaction contained 15 µl master mix plus 5 µl DNA template.

The reaction was run on a C1000 Touch Thermal Cycler from Bio-Rad following the temperature profile as described in Tab. 4.

**Tab. 4: PCR temperature profile.**

| Temperature | Time | Cycles |
|---|---|---|
| 95 °C | 10 min | |
| 94 °C | 30 s | 45 cycles with ramp rate 2.5 °C / s |
| 60 °C | 60 s | |
| 98 °C | 10 min | |

### 3. Test material

The experiment aimed at detecting small amounts of wildtype kernels in 3,000 kernel bulks of GM event SYN-IR162-4 (MIR162). Due to the lack of 100% pure GM seed lots of that size, 500 g of homozygous GM line 9FD0006t009 were grinded. The flour was thoroughly mixed and 24 sub samples of 10 g were drawn. Following this procedure, an even distribution of possible unintended wildtype contamination within the GM samples was expected. Next, different levels of wildtype contamination were simulated by adding small amounts of grinded wildtype kernels of line 9FD0006. Contaminations ranged from 0 to 5% and were each analyzed as blind samples in three replicates.

**Tab. 5: Amount of wildtype flour to be weighed in a 10 g sample of GM flour to represent a contamination from 1 to 150 wildtype kernels in a 3,000 GM kernel bulk.**

| Wildtype flour | Percent contamination | Representing no. of wildtype kernels in 3,000 GM |
|---|---|---|
| 0 mg | 0.00% | 0 |
| 3 mg | 0.03% | 1 |
| 6 mg | 0.07% | 2 |
| 15 mg | 0.17% | 5 |
| 21 mg | 0.23% | 7 |
| 30 mg | 0.33% | 10 |
| 90 mg | 1.00% | 30 |
| 450 mg | 5.00% | 150 |

### 4. Results

The ddPCR results of 24 GM flour samples with varying amounts of wildtype flour contamination were converted to the corresponding number of wildtype kernels detected in a seed bulk of 3,000 GM kernels. Measured and expected values deviated between 0 to 20 kernels, with more pronounced differences the higher the contamination level got. From 0 to 30 spiked wildtype kernels, the measurements were by maximum 3 kernels off, leading to a very precise detection in the low contamination segment. Measured and expected kernel numbers were correlated linearly with a coefficient of determination r² = 0.998. The correlation is visualized in Fig. 1.

**Tab. 6: Expected and measured wildtype kernels in 24 flour samples detected by ddPCR.**

| Sample | Expected WT kernels | Measured WT kernels | Deviation Measured-Expected |
|---|---|---|---|
| IMP19004_71 | 0 | 0 | 0 |
| IMP19004_64 | 0 | 0 | 0 |
| IMP19004_58 | 0 | 1 | 1 |
| IMP19004_70 | 1 | 1 | 0 |
| IMP19004_62 | 1 | 1 | 0 |
| IMP19004_54 | 1 | 2 | 1 |
| IMP19004_49 | 2 | 2 | 0 |
| IMP19004_53 | 2 | 2 | 0 |
| IMP19004_68 | 2 | 3 | 1 |
| IMP19004_57 | 5 | 4 | -1 |
| IMP19004_50 | 5 | 5 | 0 |
| IMP19004_60 | 5 | 5 | 0 |
| IMP19004_55 | 7 | 6 | -1 |
| IMP19004_72 | 7 | 7 | 0 |
| IMP19004_69 | 7 | 9 | 2 |
| IMP19004_51 | 10 | 7 | -3 |
| IMP19004_52 | 10 | 10 | 0 |
| IMP19004_65 | 10 | 11 | 1 |
| IMP19004_59 | 30 | 28 | -2 |
| IMP19004_66 | 30 | 28 | -2 |
| IMP19004_61 | 30 | 30 | 0 |
| IMP19004_67 | 150 | 130 | -20 |
| IMP19004_63 | 150 | 131 | -19 |
| IMP19004_56 | 150 | 138 | -11 |

The use of multiple wildtype sequences for primer design proved to be necessary as a polymorphism in the primer binding site was found. To assure that the assay also detects future conventional maize lines with putative other sequence variation, the breeding material should be monitored frequently by marker analysis.

Deviations of measured and expected wildtype kernels could be due to the way of sample preparation. The DNA content of any maize kernel is expected to be very similar, but the starch or protein content varies. Therefore, weighing in 10 g of GM flour and a specific amount of wildtype flour may not perfectly resemble the expected ratio of kernels. This hypothesis could be tested by analyzing actual seed batches instead of flour samples. However, the difficulty is to provide 100% clean GM seed lots of the target size. In previous experiments not shown here, 3,000 kernel bulks had various levels of background wildtype contamination, making it impossible to reliably differentiate the deliberately spiked wildtype kernels. The precision of the assay is sufficient to support the definition of thresholds for decision taking.

The demonstrated level of detection of 1 kernel in a 3,000 kernel bulk provides a 95 % confidence of finding a 0.1 % contamination in the original seed lot. This accuracy is state-of-the-art for adventitious presence (AP) testing. Consequently, seed samples analyzed for AP can efficiently be applied for a dual use of AP and trait purity analysis. The additional analysis creates value in form of an important quality measure at a low-cost increase.

## Claims

1. A method for quantifying in a batch of plant seeds comprising a genetic modification (GM) a contamination with plant seeds lacking said GM comprising the steps of:
a. providing a first set of primers and probe specific for the absence of said GM, and
b. providing a second set of primers and probe specific for the presence of said GM, and
c. providing a pool of nucleic acid molecules comprising an equal amount of nucleic acid molecules derived from each individual seed in said batch to be assessed, and
d. performing an amplification reaction using said first and second set of primers and probes on the pool of nucleic acid molecules of step c, and
e. detecting the signals generated by the amplification reactions of said first and second set of primers and probes, and
f. calculating the ratio of absence to presence signals,
thereby quantifying in said batch of plant seeds the contamination with plant seeds lacking said GM.

2. The method of claim 1, wherein the first and second set of primers are specific for the region of up to 1000 nucleotides upstream and/or downstream of the locus of said GM.

3. The method of claim 1 or 2, wherein the first set of primers and probes comprises at least two forward and/or reverse primers specific for the genomic insertion site of said GM of various plant genotypes lacking said GM.

4. The method of any of claims 1 to 3, wherein the second set of primers and probes comprises at least two probes detecting distinct variants of the GM.

5. The method of any of claims 1 to 4, wherein the probes are fluorescent probes.

6. The method of any of claims 1 to 5, wherein the GM is a transgene, an insertion, a point mutation or a deletion in the genome of a plant.

7. The method of any of claims 1 to 6, wherein the amplification reaction is a ddPCR, a qPCR or a rhAMP PCR.

8. The method of any of claim 1 to 7, wherein the batch comprises at least 100 individuals.

9. The method of any of claims 1 to 9, wherein the contamination is equal or less than 10%.

10. The method of any of claims 1 to 9, wherein a threshold of 10 seeds per 3000 lacking said GM is defined to identify seed batches to be discarded.

11. The method of any of claims 1 to 10, wherein the pool of nucleic acid molecules is used for screening for adventitious presence of undesired GM events.

12. A composition for quantifying in batch of plant seeds comprising a nucleotide sequence of interest (NOI) the occurrence of plant seeds lacking said NOI comprising
a. a first set of primers and probe specific for the absence of the NOI, and
b. a second set of primers and probe specific for the NOI, and
c. a pool of nucleic acid molecules comprising an equal amount of nucleic acid molecules derived from each individual in said population or batch to be assessed.

13. The composition of claim 12, wherein the first and second set of primers are specific for the region of up to 1000 nucleotides upstream and/or downstream of the locus of said GM.

14. The composition of claims 12 or 13, wherein the first set of primers and probes comprises at least two forward and/or reverse primers specific for the genome of various microorganisms, plants, plant seeds, tissues and/or cells lacking said NOI.

15. The composition of any of claims 12 to 14, wherein the second set of primers and probes comprises at least two probes detecting various variants of the NOI.

16. The composition of any of claims 12 to 15, wherein the probes are fluorescent probes.
